(19) Europäisches Patentamt

European Patent Office

Office européen des brevets

(11) **EP 1 642 564 A2**

(12) **DEMANDE DE BREVET EUROPEEN**

(43) Date de publication:
**05.04.2006 Bulletin 2006/14**

(51) Int Cl.:
***A61K 8/18*** (2006.01)

(21) Numéro de dépôt: **05291945.3**

(22) Date de dépôt: **08.06.1999**

(84) Etats contractants désignés:
**DE ES FR GB IE IT**

(30) Priorité: **11.06.1998 FR 9807376**

(62) Numéro(s) de document de la (des) demande(s) initiale(s) en application de l'article 76 CBE:
**99923692.0 / 1 083 867**

(71) Demandeur: **L'OREAL**
**75008 Paris (FR)**

(72) Inventeurs:
• **Rollat-Corvol, Isabelle**
**75017 Paris (FR)**

• **Samain, Henri**
**91570 Bievres (FR)**

(74) Mandataire: **Bourdeau, Françoise**
**L'OREAL - D.I.P.I.**
**25-29 Quai Aulagnier**
**92600 Asnières (FR)**

Remarques:
Cette demande a été déposée le 20 - 09 - 2005 comme demande divisionnaire de la demande mentionnée sous le code INID 62.

(54) **Composition cosmétique comprenant au moins un polymère collant et au moins un polymère fixant**

(57) L'invention a pour objet une composition cosmétique pour les fibres kératiniques telles que les cheveux comprenant, dans un milieu cosmétiquement acceptable, au moins un polymère collant de température de transition vitreuse (Tg) inférieure à 20°C et au moins un polymère fixant de température de transition vitreuse (Tg) supérieure à 15°C. Elle vise également un procédé de traitement des fibres kératiniques telles que les cheveux, en particulier un procédé de fixation et/ou de maintien de la coiffure, mettant en oeuvre ladite composition ainsi que l'utilisation de cette composition dans ou pour la fabrication d'une formulation cosmétique de coiffage.

EP 1 642 564 A2

**EP 1 642 564 A2**

## Description

L'invention a pour objet une composition cosmétique pour les fibres kératiniques telles que les cheveux comprenant, dans un milieu cosmétiquement acceptable, au moins un polymère collant de température de transition vitreuse inférieure à 20°C et au moins un polymère fixant de température de transition vitreuse supérieure à 15°C. Elle vise également un procédé de traitement des fibres kératiniques telles que les cheveux, en particulier un procédé de fixation et/ou de maintien de la coiffure, mettant en oeuvre ladite composition ainsi que l'utilisation de cette composition dans ou pour la fabrication d'une formulation cosmétique de coiffage.

Au sens de la présente invention, on entend par "fibres kératiniques", les cheveux, les cils et les sourcils et par "polymère collant", un polymère qui, après application par pression sur un polymère identique, résiste à une tentative de séparation.

La fixation de la coiffure est un élément important du coiffage qui consiste à maintenir la mise en forme déjà réalisée ou à mettre en forme les cheveux et à les fixer simultanément. On entend par "polymère fixant", un polymère qui maintient en forme les cheveux ou qui permet de mettre en forme les cheveux et de les fixer simultanément.

Les produits capillaires pour la mise en forme et/ou le maintien de la coiffure les plus répandus sur le marché de la cosmétique sont des compositions à pulvériser essentiellement constituées d'une solution le plus souvent alcoolique ou aqueuse et d'un ou plusieurs matériaux, généralement des résines polymères, dont la fonction est de former des soudures entre les cheveux, appelés encore matériaux fixants, en mélange avec divers adjuvants cosmétiques. Cette solution est généralement conditionnée soit dans un récipient aérosol approprié mis sous pression à l'aide d'un propulseur, soit dans un flacon pompe.

On connaît également les gels ou les mousses de coiffage qui sont généralement appliqués sur les cheveux mouillés avant de faire un brushing ou une mise en plis. A la différence des laques aérosols classiques, ces compositions présentent l'inconvénient de ne pas permettre la fixation des cheveux dans une forme déjà réalisée. En effet, ces compositions sont essentiellement aqueuses et leur application mouille les cheveux et ne peut donc maintenir la forme initiale de la coiffure. Pour mettre en forme et fixer la coiffure, on doit donc ensuite effectuer un brushing ou un séchage.

La plupart des compositions de l'état de la technique présentent le même inconvénient de ne pas fixer ou maintenir la coiffure suffisamment durablement. Ainsi, la forme donnée initialement à la coiffure s'estompe progressivement au cours de la journée, et ceci d'autant plus vite d'ailleurs que la personne est en mouvement. En conséquence, il est souvent nécessaire de recommencer l'ensemble des opérations de coiffage et de fixation si l'on souhaite retrouver la coiffure initiale.

On recherche donc des compositions de coiffage qui procurent un effet de fixation et de maintien suffisamment forts pour que la coiffure résiste convenablement dans le temps aux diverses sollicitations.

Enfin, les compositions destinées à la fixation de la coiffure présentent parfois l'inconvénient d'altérer les propriétés cosmétiques des cheveux. Ainsi, les cheveux peuvent devenir rêches, difficiles à démêler, perdre leur toucher et leur aspect agréables. On recherche donc des compositions coiffantes procurant de bonnes propriétés cosmétiques, notamment en terme de démêlage, de douceur et de toucher.

Il existe donc un besoin de trouver des compositions cosmétiques, notamment pour le coiffage, qui ne présentent pas l'ensemble des inconvénients indiqués ci-dessus.

De manière surprenante et inattendue, la Demanderesse a découvert que lorsque l'on associe des polymères collants, en particulier des polyesters sulfoniques ramifiés ou des polymères d'ester (méth)-acrylique, avec certains polymères fixants, il est possible d'obtenir des compositions cosmétiques répondant aux exigences exprimées ci-dessus.

L'invention a donc pour objet une composition cosmétique pour les fibres kératiniques telles que les cheveux comprenant, dans un milieu cosmétiquement acceptable, au moins un polymère collant de température de transition vitreuse (Tg) inférieure à 20°C et au moins un polymère fixant de température de transition vitreuse(Tg) supérieure à 15°C.

De manière avantageuse, on choisit un polymère collant présentant un profil de décollement défini par au moins une force maximale de décollement $F_{max}$ > 3 Newton, et de préférence supérieure à 5N.

Plus avantageusement encore, le profil de décollement est défini en outre par une énergie de séparation $E_{s(M/V)}$ du matériau mis en contact avec une surface en verre, inférieure à 300 µJ, lorsque la température de transition vitreuse du polymère collant est inférieure à -15°C.

La force maximale de décollement Fmax est la force maximale de traction, mesurée à l'aide d'un extensomètre, nécessaire pour décoller les surfaces de 38 mm$^2$, respectives de deux supports (A) et (B), rigides, inertes, non-absorbants, placés en regard l'un de l'autre ; lesdites surfaces étant enduites préalablement par le polymère collant préalablement dissous à 5% dans un solvant aqueux, hydroalcoolique ou alcoolique, à raison de 1 mg/mm$^2$ , séchées pendant 24 heures à 22°C sous une humidité relative de 50%, puis soumises pendant 20 secondes à une compression de 3 Newton et enfin soumises pendant 30 secondes à une traction de vitesse 20 mm/minute.

Avantageusement, on utilise des supports (A) et (B) constitués de polyéthylène, de polypropylène, d'alliage métallique ou de verre.

L'énergie de séparation $E_{s(M/V)}$ est l'énergie fournie par l'extensomètre pour effectuer la séparation des surfaces

2

respectives de 38 mm$^2$, de deux supports (C) et (D), rigides, inertes, non absorbants et placés en regard l'un de l'autre ; l'un desdits supports étant constitué de verre poli et l'autre desdits supports étant de nature identique à celle des supports (A) et (B) tels que définis dans la revendication 2 ou 3 et dont la surface est enduite préalablement par le polymère collant préalablement dissous à 5% dans un solvant aqueux, hydroalcoolique ou alcoolique, à raison de 1 mg/mm$^2$, séchées pendant 24 heures à 22°C sous une humidité relative de 50%, les deux surfaces desdits supports (C) et (D) étant soumises pendant 20 secondes à une compression de 3 Newton et enfin soumises pendant 30 secondes à une traction de vitesse 20 mm/minute.

L'énergie de séparation $E_{s(M/V)}$ est un travail qui peut être calculé au moyen de la formule suivante :

$$\int_{Xs1 + 0,05}^{Xs2} F(x)dx$$

où F(x) est la force nécessaire pour produire un déplacement (x) ;
$x_{s1}$ est le déplacement (exprimé en millimètres) produit par la force de traction maximale ;
$X_{s2}$ où le déplacement (exprimé en millimètres) produit par la force de traction permettant la séparation totale des deux surfaces des supports (C) et (D).

De préférence, on choisira un polymère collant tel que la force maximale de décollement $F_{max}$ soit supérieure à 5 Newton et/ou tel que sa température de transition vitreuse (Tg) soit inférieure à 20°C. Si la Tg du polymère est inférieure à - 15°C, il devra préférentiellement avoir en plus une énergie de séparation $E_{s(M/V)}$ inférieure à 300 µJ.

La concentration relative en poids en polymère collant dans la composition est en général supérieure à 0,01 %, plus préférentiellement supérieure à 0,1%, et plus préférentiellement encore supérieure à 0,5%.

Selon un premier mode de réalisation avantageux de la présente invention, on choisit, comme polymère collant, un polymère sulfonique ramifié ou un polymère polymères d'ester (méth)-acrylique.

Avantageusement, on choisit un polymère fixant qui présente une température de transition vitreuse (Tg) supérieure à 25°C.

Conformément à l'invention, la concentration relative en poids en polymère fixant dans la composition est en général supérieure à 0,01 %, et de préférence supérieure à 0,1%.

Une forme particulièrement préférée du polyester sulfonique ramifié est celle obtenue par polymérisation de:

(i) au moins un acide dicarboxylique difonctionnel ne portant pas de fonction sulfonique;
(ii) au moins un monomère difonctionnel portant au moins une fonction sulfonique, le ou les groupements fonctionnels étant choisis dans le groupe comprenant les groupements hydroxyle, carboxyle et amino;
(iii) au moins un diol ou un mélange de diol(s) et de diamine(s);
(iv) éventuellement un monomère difonctionnel choisi dans le groupe comprenant les hydroxyacides carboxyliques, les aminoacides carboxyliques et leurs mélanges;
(v) au moins un réactif multifonctionnel portant au moins trois groupements fonctionnels choisis dans le groupe comprenant les groupements amino, alcool et acide carboxylique.

Une telle polymérisation peut être effectuée à partir de:

(i) au moins un acide dicarboxylique difonctionnel ne portant pas de fonction sulfonique;
(ii) 2 à 15 % relatif en mole de monomère difonctionnel portant au moins une fonction sulfonique;
(iii) au moins un diol ou un mélange de diol (s) et de diamine (s);
(iv) 0 à 40 % relatif en mole du monomère difonctionnel choisi dans le groupe comprenant les hydroxyacides carboxyliques, les aminoacides carboxyliques et leurs mélanges;
(v) 0,1 à 40 % relatif en mole du réactif multifonctionnel portant au moins trois groupements fonctionnels réactifs.

Les polymères sulfoniques ramifiés contiennent de préférence des proportions substantiellement égales, en nombre d'équivalents, d'une part, en fonctions acide carboxylique et d'autre part, en fonctions diol et/ou diol et diamine.

L'acide dicarboxylique difonctionnel (i) est de préférence choisi dans le groupe comprenant les acides aliphatiques dicarboxyliques, les acides alicycliques dicarboxyliques, des acides aromatiques dicarboxyliques ou un mélange de ceux-ci et plus particulièrement dans le groupe comprenant l'acide 1,4 - cyclohexanedioïque, l'acide succinique, l'acide glutarique, l'acide adipique, l'acide azelaïque, acide sebacique, l'acide fumarique, l'acide maléïque, l'acide 1,3 - cyclohexanedioïque, l'acide phtalique, l'acide téréphtaltique et l'acide isophtalique ou leurs mélanges.

Le monomère difonctionnel (ii) tel que défini ci-dessus est de préférence choisi dans le groupe comprenant les acides dicarboxyliques, les esters d'acide dicarboxylique, les glycols et les hydroxyacides contenant, chacun, au moins un groupement métal sulfonate.

Le diol (iii) est de préférence choisi dans le groupe comprenant les alcanediols et les polyalkylènediols et plus particulièrement dans le groupe comprenant l'éthylène glycol, le propylène glycol, le diéthylène glycol, le triéthylène glycol et le polyproprylène glycol.

La diamine (iii) peut être choisie dans le groupe comprenant les alcanediamines et les polyalkylènediamines.

Le réactif multifonctionnel (v) est choisi de préférence dans le groupe comprenant le triméthyloléthane, le triméthylolpropane, le glycérol, le pentaérythritol, le sorbitol, l'anhydride trimellitique, l'érythritol, le threitol, le dipentaérythritol, le dianhydride pyromellitique et l'acide diméthylpropionique.

Les polymères sulfoniques ramifiés particulièrement visés par la présente invention sont ceux décrits dans les demandes de brevets WO 95/181 91, WO 97/082 61 et WO 97/208 99.

Conformément à l'invention, on choisit avantageusement, comme polymère sulfonique ramifié, le polymère AQ 1350 commercialisé par la Société Eastman. Ce polymère AQ 1350 est défini par:

- une température de transition vitreuse donnée par le fournisseur égale à 0°C;
- une force maximale de décollement Fmax égale à 25N.

Selon un deuxième mode de réalisation avantageux des compositions conformes à l'invention, on utilise, comme polymère collant, un polymère d'ester (méth)-acrylique.

Les polymères collants d'ester (méth)-acrylique utilisés conformément à l'invention comprennent avantageusement:

(a) de 9 à 99% en poids d'un monomère d'ester (méth)-acrylique par rapport au poids total du polymère;
(b) jusqu'à 90% de comonomère(s);
(c) de 1 à 10 % d'un monomère vinylidène contenant un groupement carboxyle ou hydroxyle.

Le monomère d'ester (méth)-acrylique (a) répond généralement à la formule (I) ou (II):

CH2=CH-COOR (I)

CH2=C(CH3)-COOR (II)

dans lesquelles R représente un alkyle en $C_1$ à $C_{18}$, un alkoxyalkyle en $C_2$ à $C_8$, un alkylthioalkyle en $C_2$ à $C_8$ ou un cyanoalkyle en $C_2$ à $C_8$. A titre d'exemple, le monomère (a) peut être choisi dans le groupe comprenant l'acrylate de méthyle, l'acrylate d'éthyle, l'acrylate de n-butyle, l'acrylate d'isobutyle, l'acrylate d'hexyle, l'acrylate d'octyle, l'acrylate de 2-éthylhexyle, l'acrylate de décyle, le méthoxyacrylate, l'éthoxyacrylate, l'acrylate de méthylthiométhyle et l'acrylate de cyanopropyle.

Le comonomère (b) peut contenir un ou plusieurs groupes vinylidène ayant des groupes CH2=C terminaux, tels que:

- les esters acryliques ou méthacryliques, comme le méthacrylate de méthyle, le méthacrylate d'éthyle, le méthacrylate de n-butyle, l'éthacrylate de méthyle,
- les halogénures de vinyle tel que le chlorure de vinyle;
- les esters de vinyle et d'allyle tels que l'acétate de vinyle, le butyrate de vinyle, le chloroacétate de vinyle;
- les vinyles aromatiques tels que le styrène, le vinyltoluène, le chlorométhylstyrène, le vinylnaphtalène; et
- les nitriles vinyliques tels que l'acrylonitrile ou le méthacrylonitrile.

Parmi les monomères vinylidène contenant des groupement hydroxyles (c), on peut citer les monomères acrylates à groupement hydroxyle terminal, tel que l'hydroxyéthyle acrylate, l'hydroxyéthyle méthacrylate, l'hydroxypropyle acrylate, l'hydroxyéthyle méthacrylate, l'hydroxybuthyle acrylate ou encore certains dérivés hydroxyméthylés d'acrylamide diacétone, par exemple, le N-méthylol acrylamide, le N-méthylol maléamide, le N-propanolacrylamide, le N-méthylol méthacrylamide, le N-méthylol-p-vinyl benzamide.

Parmi les monomères vinylidène contenant des groupement carboxyles (c), on peut citer par exemple l'acide acrylique ou méthacrylique, l'acide itaconique, l'acide citraconique, l'acide maléique.

Les polymères collants d'ester (méth)-acrylique particulièrement visés par la présente invention sont ceux décrits dans les brevets US 5 234 627 et US 4 007 147.

Conformément à l'invention, on choisit avantageusement comme polymère polymères d'ester (méth)-acrylique, le polymère Hycar 26 120 commercialisé par la Société Goodrich. Ce polymère Hycar 26 120 est défini par:

- une température de transition vitreuse donnée par le fournisseur égale à -10°C;
- une force maximale de décollement Fmax égale à 6,25N.

Le polymère fixant est généralement choisi parmi les polymères fixants anionique, cationique, amphotère, non ionique et leurs mélanges.

Ces polymères fixants peuvent être utilisés sous forme solubilisée ou encore sous forme de dispersion de particules solides de polymère.

En tant que polymère fixant cationique, on choisit préférentiellement les polymères comportant des groupements amine primaire, secondaire, tertiaire et/ou quaternaire faisant partie de la chaîne polymère ou directement reliés à celle-ci, et ayant un poids moléculaire compris entre 500 et environ 5.000.000 et de préférence entre 1000 et 3.000.000.

En tant que polymères fixants anioniques, on préfère les polymères comportant des groupements dérivés d'acide carboxylique, sulfonique ou phosphorique et ont un poids moléculaire moyen en poids compris entre environ 500 et 5.000.000.

En tant que polymères fixants amphotères, on choisit préférentiellement les polymères comportant des motifs B et C répartis statistiquement dans la chaîne polymère, où B désigne un motif dérivant d'un monomère comportant au moins une fonction basique, en particulier un atome d'azote basique et C désigne un motif dérivant d'un monomère acide comportant un ou plusieurs groupements carboxyliques ou sulfoniques ou bien B et C peuvent désigner des groupements dérivant de monomères zwittérioniques de carboxybétaïnes ou de sulfobétaïnes; B et C peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné; ou bien B et C font partie d'une chaîne d'un polymère à motif éthylène $\alpha$, $\beta$-dicarboxylique dont l'un des groupements carboxyliques a été amené à réagir avec une polyamine comportant un ou plusieurs groupements amine primaire ou secondaire.

En tant que polymères fixants non ioniques, on choisit avantageusement les polyuréthannes.

Parmi les polymères fixants utilisés sous forme solubilisée, on utilisera de préférence les polymères choisis dans le groupe comprenant les polymères acryliques siliconés, les polymères à base de monomère vinyl pyrrolidone et vinyl caprolactame.

Parmi les polymères fixants se présentant sous la forme d'une dispersion, on utilisera de préférence ceux comprenant des monomères acryliques ou métacryliques et leurs esters ou encore ceux comprenant des monomères styrène.

La composition peut se présenter sous forme vaporisable, de mousse, de gel ou de lotion et le véhicule cosmétiquement acceptable peut être constitué par un solvant approprié, auquel sont ajoutés des additifs tels que des agents gélifiants ou des agents moussants. En général, le solvant est choisi parmi l'eau, les alcools ou un mélange hydroalcoolique.

Les compositions peuvent contenir, en outre, une quantité appropriée de propulseurs tels que des gaz comprimés ou liquéfiés usuels, de préférence l'air, le gaz carbonique ou l'azote comprimés, ou encore un gaz soluble ou non dans la composition tel que le diméthyl éther, les hydrocarbures fluorés ou non et leurs mélanges.

L'invention a également pour objet un dispositif aérosol constitué par un récipient contenant une composition aérosol constituée par d'une part une phase liquide (ou jus) contenant au moins une composition conforme à l'invention dans un solvant approprié et un propulseur ainsi qu'un moyen de distribution de ladite composition aérosol.

Encore un autre objet de l'invention est un procédé de traitement des fibres kératiniques, en particulier des cheveux, caractérisé en ce qu'on applique sur lesdites fibres la composition conforme à l'invention, avant ou après la mise en forme de la coiffure.

La composition conforme à l'invention est généralement utilisée dans ou pour la fabrication d'une formulation cosmétique de coiffage.

Les exemples ci-après permettent d'illustrer l'invention sans toutefois chercher à en limiter la portée. On utilisera les polymères indiqués ci-après:

| | |
|---|---|
| Amphomer | Copolymère octylacrylamide /acrylate/ butylaminoéthyl/méthacrylate commercialisé par National Starch |
| Polymer LO-21 DRY | Poly diméthyl / méthyl siloxane à groupements propyl thio-3 acrylate de méthyle / méthacrylate de méthyle / acide méthacrylique commercialisé par 3M |
| Luviskol VA64P | Polyvinylpyrrolidone commercialisé par BASF |
| Uramul SC 132 | Latex copolymère acrylique commercialisé par DMS RESINS; Tg = 50°C |
| AQ 1350 | Polyester sulfonique ramifié commercialisé par la Société Eastman |

EXEMPLES:

On compare ci-après des compositions conformes à l'invention comprenant une association d'un polymère sulfonique ramifié et d'un polymère fixant avec des compositions conformes à l'art antérieur contenant soit le polymère sulfonique ramifié seul, soit le polymère fixant seul.

Exemple 1 (comparatif):

On réalise des tests sensoriels pour comparer la performance de compositions conformes à l'invention et de compositions conformes à l'art antérieur. La comparaison porte sur la tenue dans le temps et sous contrainte de la coiffure.
Pour cela, on réalise 3 compositions conformes à l'invention et 4 compositions conformes à l'art antérieur. On applique ces compositions sur des perruques de cheveux naturels. Puis, on évalue la tenue de la forme de la perruque et le retour de la forme des perruques après agitation.

Composition 1 (invention):

| | |
|---|---|
| AQ 1350 | 4 g |
| Amphomer | 2 g |
| Eau | 75 g |
| 2 Amino-2-méthyl-1-propanol qs neutralisation Amphomer | 0,37 g |
| Alcool qs | 100 g |

Composition 2 (invention):

| | |
|---|---|
| AQ 1350 | 4 g |
| Polymère LO-21 DRY préalablement neutralisé à 90 % | 2 g |
| Eau | 75 g |
| Alcool qs | 100 g |

Composition 3 (invention):

| | |
|---|---|
| AQ 1350 | 4 g |
| LUVISKOL VA 64 P | 2 g |
| Eau | 75 g |
| Alcool qs | 100 g |

Composition 4 (art antérieur- polyester sulfonique ramifié seul):

| | |
|---|---|
| AQ 1350 | 6 g |
| Eau | 75 g |
| Alcool qs | 100 g |

Composition 5 (art antérieur- polymère fixant seul):

| | |
|---|---|
| Amphomer | 6 g |
| Eau | 75 g |
| 2 Amino 2 méthyl 1 propanol qs neutralisation Amphomer | 1,09 g |
| Alcool qs | 100 g |

Composition 6 (art antérieur- polymère fixant seul):

| | |
|---|---|
| Polymer LO-21 DRY préalablement neutralisé à 90% | 6 g |
| Eau | 75 g |
| Alcool qs | 100 g |

Composition 7 (art antérieur- polymère fixant seul):

| | |
|---|---|
| LUVISKOL VA 64 P | 6 g |
| Eau | 75 g |
| Alcool qs | 100 g |

On introduit chacune des compositions dans un flacon pompe. On pulvérise 3 grammes de chaque composition sur une perruque de cheveux de 20 cm de longueur préalablement shampooinée et essorée. On laisse sécher pendant 4 heures et on retourne la perruque.

On agite la perruque au moyen d'une rotation alternative pendant 2 heures. On compare la forme finale de la chevelure avec la forme qu'elle avait avant agitation et on estime la tenue de la forme. On utilise la notation de 0 à 5:

- 0 traduit une très mauvaise tenue de la forme et une coiffure entièrement affaissée.
- 5 traduit une excellente tenue et une coiffure restée intacte et volumineuse en dépit de l'agitation.
  On démêle ensuite les perruques et on les secoue à nouveau pendant 20 secondes. On estime le retour de la forme de la coiffure lorsqu'elle a subi toutes ces opérations. On utilise la même grille de notation allant de 0 à 5.

Le tableau 1 résume les résultats.

Tableau 1

| Composition | Tenue de la forme après agitation | Retour de la forme après agitation et démêlage |
|---|---|---|
| 1 | 3,25 | 2,5 |
| 2 | 4,0 | 4,0 |
| 3 | 4,5 | 4,25 |
| 4 | 2,0 | 4,0 |
| 5 | 3,75 | 0,75 |
| 6 | 3,5 | 1,0 |
| 7 | 2,0 | 0,75 |
| sans traitement | 0 | 0,5 |

Le tableau 1 montre que les compositions conformes à l'invention et comprenant l'association de polymères procurent de meilleurs résultats en terme de tenue de la forme après agitation et de retour de la forme après agitation et démêlage que les compositions conformes à l'art antérieur.

Exemple 2:

On réalise une composition 8 conforme à l'invention et on estime la tenue de la coiffure ainsi que certaines propriétés cosmétiques.

Composition 8 (invention):

| | |
|---|---|
| AQ 1350 | 4g |
| URAMUL SC 132 | 0,5 g |
| Eau qs | 100 g |

On prend une perruque de 20 g de cheveux naturels, on applique 2,5 grammes de la composition 8 sur les cheveux et on laisse sécher.

On observe que les cheveux présentent un très bon maintien. Le démêlage est facile et la chevelure présente un bon toucher après démêlage.

**Revendications**

1. Composition cosmétique pour les fibres kératiniques telles que les cheveux, **caractérisée par le fait qu'**elle comprend, dans un milieu cosmétiquement acceptable, au moins un polymère collant de température de transition vitreuse (Tg) inférieure à 20°C et au moins un polymère fixant de température de transition vitreuse (Tg) supérieure à 15°C.

2. Composition selon la revendication 1, **caractérisée par le fait que** le polymère collant présente un profil de décollement défini par au moins une force maximale de décollement $F_{max}$ > 3 Newton, et de préférence supérieure à 5N.

3. Composition selon la revendication 2, **caractérisée par le fait que**, lorsque la température de transition vitreuse du polymère collant est inférieure à -15°C, le profil de décollement est en outre défini par une énergie de séparation $E_{s(M/V)}$ du matériau mis en contact avec une surface en verre, inférieure à 300 µJ.

4. Composition selon la revendication 2, **caractérisée par le fait que** Fmax est la force maximale de traction, mesurée à l'aide d'un extensomètre, nécessaire pour décoller les surfaces de 38 mm$^2$, respectives de deux supports (A) et (B), rigides, inertes, non absorbants, placés en regard l'un de l'autre ; lesdites surfaces étant enduites préalablement par le polymère collant préalablement dissous à 5% dans un solvant aqueux, hydroalcoolique ou alcoolique, à raison de 1 mg/mm$^2$, séchées pendant 24 heures à 22°C sous une humidité relative de 50 %, puis soumises pendant 20 secondes à une compression de 3 Newton et enfin soumises pendant 30 secondes à une traction de vitesse 20 mm/minute.

5. Composition selon la revendication 4, **caractérisée par le fait que** les supports (A) et (B) sont constitués de polyéthylène, de polypropylène, d'alliage métallique ou de verre.

6. Composition selon la revendication 2, **caractérisée par le fait que** $E_{s(M/V)}$ est l'énergie fournie par l'extensomètre pour effectuer la séparation des surfaces respectives de 38 mm$^2$, de deux supports (C) et (D), rigides, inertes, non absorbants et placés en regard l'un de l'autre ; l'un desdits supports étant constitué de verre poli et l'autre desdits supports étant de nature identique à celle des supports (A) et (B) tels que définis dans la revendication 4 ou 5 et dont la surface est enduite préalablement par le polymère collant préalablement dissous à 5% dans un solvant aqueux, hydroalcoolique ou alcoolique, à raison de 1 mg/mm$^2$, séchées pendant 24 heures à 22°C sous une humidité relative de 50%, les deux surfaces desdits supports (C) et (D) étant soumises pendant 20 secondes à une compression de 3 Newton et enfin soumises pendant 30 secondes à une traction de vitesse 20 mm/minute.

7. Composition selon la revendication 6, **caractérisée par le fait que** $E_{s(M/V)}$ est le travail calculé au moyen de la formule suivante :

$$\int_{X_{s1} + 0,05}^{X_{s2}} F(x)dx$$

où F(x) est la force nécessaire pour produire un déplacement (x) ;
$x_{s1}$ est le déplacement (exprimé en millimètres) produit par la force de traction maximale ;
$X_{s2}$ où le déplacement (exprimé en millimètres) produit par la force de traction permettant la séparation totale des deux surfaces des supports (C) et (D) .

8. Composition selon lune quelconque des revendications précédentes, **caractérisée par le fait que** le polymère collant est un polyester sulfonique ramifié ou un polymère polymères d'ester (méth)-acrylique.

9. Composition selon l'une quelconque des revendications précédentes, **caractérisée par le fait que** la concentration relative en poids en polymère collant dans la composition est supérieure à 0,01 %, de préférence supérieure à 0,1%, et plus préférentiellement encore supérieure à 0,5 %.

10. Composition selon l'une quelconque des revendications précédentes, **caractérisée par le fait que** le polymère fixant présente une température de transition vitreuse (Tg) supérieure à 25°C.

11. Composition selon l'une quelconque des revendications précédentes, **caractérisée par le fait que** la concentration relative en poids en polymère fixant dans la composition est supérieure à 0,01 %, et de préférence supérieure à 0,1 %.

**12.** Composition selon la revendication 8, **caractérisée par le fait que** le polyester sulfonique ramifié est formé par polymérisation de:

(i) au moins un acide dicarboxylique difonctionnel ne portant pas de fonction sulfonique;
(ii) au moins un monomère difonctionnel portant au moins une fonction sulfonique, le ou les groupements fonctionnels étant choisis dans le groupe comprenant les groupements hydroxyle, carboxyle et amino;
(iii) au moins un diol ou un mélange de diol (s) et de diamine (s);
(iv) éventuellement un monomère difonctionnel choisi dans le groupe comprenant les hydroxyacides carboxyliques, les aminoacides carboxyliques et leurs mélanges;
(v) au moins un réactif multifonctionnel portant au moins trois groupements fonctionnels choisis dans le groupe comprenant les groupements amino, alcool et acide carboxylique.

**13.** Composition selon la revendication 12, **caractérisée par le fait que** la polymérisation est effectuée à partir de:

(i) au moins un acide dicarboxylique difonctionnel ne portant pas de fonction sulfonique;
(ii) 2 à 15 % relatif en mole de monomère difonctionnel portant au moins une fonction sulfonique;
(iii) au moins un diol ou un mélange de diol (s) et de diamine (s);
(iv) 0 à 40 % relatif en mole du monomère difonctionnel choisi dans le groupe comprenant les hydroxyacides carboxyliques, les aminoacides carboxyliques et leurs mélanges;
(v) 0,1 à 40 % relatif en mole du réactif multifonctionnel portant au moins trois groupements fonctionnels réactifs.

**14.** Composition selon l'une quelconque des revendications 12 ou 13, **caractérisée par le fait que** le polymère sulfonique ramifié contient des proportions substantiellement égales, en nombre d'équivalents, d'une part de fonctions acide carboxylique et d'autre part de fonctions diol et/ou diol et diamine.

**15.** Composition selon l'une quelconque des revendications 12 à 14, **caractérisée par le fait que** l'acide dicarboxylique difonctionnel (i) est choisi dans le groupe comprenant les acides aliphatiques dicarboxyliques, les acides alicycliques dicarboxyliques, des acides aromatiques dicarboxyliques.

**16.** Composition selon la revendication 15, **caractérisée par le fait que** l'acide dicarboxylique difonctionnel (i) est choisi dans le groupe comprenant l'acide 1,4 - cyclohexanedioïque, l'acide succinique, l'acide glutarique, l'acide adipique, l'acide azelaïque, acide sebacique, l'acide fumarique, l'acide maléïque, l'acide 1,3 - cyclohexanedioïque, l'acide phtalique, l'acide téréphtaltique et l'acide isophtalique et un mélange de ceux-ci.

**17.** Composition selon l'une quelconque des revendications 12 à 14, **caractérisée par le fait que** le monomère difonctionnel (ii) est choisi dans le groupe comprenant les acides dicarboxyliques, les esters d'acide dicarboxylique, les glycols et les hydroxyacides contenant, chacun, au moins un groupement métal sulfonate.

**18.** Composition selon l'une quelconque des revendications 12 à 14, **caractérisée par le fait que** le diol (iii) est choisi dans le groupe comprenant les alcanediols et les polyalkylènediols.

**19.** Composition selon la revendication 18, **caractérisée par le fait que** le diol (iii) est choisi dans le groupe comprenant l'éthylène glycol, le propylène glycol, le diéthylène glycol, le triéthylène glycol et le polyproprylène glycol.

**20.** Composition selon l'une quelconque des revendications 12 à 14, **caractérisée par le fait que** la diamine (iii) est choisie dans le groupe comprenant les alcanediamines et les polyalkylènediamines.

**21.** Composition selon l'une quelconque des revendications 12 à 14, **caractérisée par le fait que** le réactif multifonctionnel (v) est choisi dans le groupe comprenant le triméthyloléthane, le triméthylolpropane, le glycérol, le pentaérythritol, le sorbitol, l'anhydride trimellitique, l'érythritol, le threitol, le dipentaérythritol, le dianhydride pyromellitique et l'acide diméthylpropionique.

**22.** Composition selon la revendication 8, **caractérisée par le fait que** le polymère d'ester (méth)-acrylique comprend avantageusement:

(a) de 9 à 99% en poids d'un monomère d'ester (méth)-acrylique par rapport au poids total du polymère;

(b) jusqu'à 90% de comonomère;

(c) de 1 à 10 % d'un monomère vinylidène contenant un groupement carboxyle ou hydroxyle.

23. Composition selon l'une quelconque des revendications précédentes, **caractérisée en ce que** le polymère fixant est choisi parmi les polymères fixants anionique, cationique, amphotère, non ionique et leurs mélanges.

24. Composition selon la revendication 23, **caractérisée par le fait que** les polymères fixants se présentent sous forme solubilisée ou sous forme de dispersion de particules solides de polymère.

25. Composition selon la revendication 24, **caractérisée en ce que** les polymères fixants cationiques sont choisis parmi les polymères comportant des groupements amine primaire, secondaire, tertiaire et/ou quaternaire faisant partie de la chaîne polymère ou directement reliés à celle-ci, et ayant un poids moléculaire compris entre 500 et environ 5.000.000 et de préférence entre 1000 et 3.000.000.

26. Composition selon la revendication 23, **caractérisée en ce que** les polymères fixants anioniques sont des polymères comportant des groupements dérivés d'acide carboxylique, sulfonique ou phosphorique et ont un poids moléculaire moyen en poids compris entre environ 500 et 5.000.000.

27. Composition selon la revendication 23, **caractérisée par le fait que** les polymères fixants sont des polymères amphotères choisis parmi les polymères comportant des motifs B et C répartis statistiquement dans la chaîne polymère, où B désigne un motif dérivant d'un monomère comportant au moins une fonction basique, en particulier un atome d'azote basique et C désigne un motif dérivant d'un monomère acide comportant un ou plusieurs groupements carboxyliques ou sulfoniques ou bien B et C peuvent désigner des groupements dérivant de monomères zwittérioniques de carboxybétaïnes ou de sulfobétaïnes; B et C peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné; ou bien B et C font partie d'une chaîne d'un polymère à motif éthylène α,β-dicarboxylique dont l'un des groupements carboxyliques a été amené à réagir avec une polyamine comportant un ou plusieurs groupements amine primaire ou secondaire.

28. Composition selon la revendication 23, **caractérisée en ce que** les polymères fixants non ioniques sont des polyuréthannes.

29. Composition selon la revendication 1, **caractérisée par le fait que** le polymère fixant est un polymère hydrosoluble choisi dans le groupe comprenant les polymères acryliques siliconés, les polymères à base de monomère vinyl pyrrolidone et vinyl caprolactame.

30. Composition selon la revendication 1, **caractérisée par le fait que** le polymère fixant est un polymère dispersé à base de monomères acryliques ou méthacryliques et leurs esters et un polymères à base de monomères styrène.

31. Composition selon l'une quelconque des revendications précédentes, **caractérisée en ce qu'**elle se présente sous la forme d'une composition vaporisable, de mousse, de gel ou de lotion.

32. Composition selon l'une quelconque des revendications précédentes, **caractérisée en ce que** le véhicule cosmétiquement acceptable est constitué par un solvant approprié, auquel peuvent être ajoutés des additifs tels que des agents gélifiants ou des agents moussants.

33. Composition selon l'une quelconque des revendications précédentes, **caractérisée en ce qu'**elle comprend un solvant choisi parmi l'eau, un alcool ou un mélange hydroalcoolique.

34. Composition selon l'une quelconque des revendications précédentes, **caractérisée en ce qu'**elle comprend en outre une quantité appropriée de propulseur constitué par les gaz comprimés ou liquéfiés usuels, de préférence l'air, le gaz carbonique ou l'azote comprimés, ou encore un gaz soluble ou non dans la composition tel que le diméthyl éther, les hydrocarbures fluorés ou non, et leurs mélanges.

35. Dispositif aérosol constitué par un récipient contenant une composition aérosol constituée par d'une part une phase liquide (ou jus) contenant au moins une composition selon l'une quelconque des revendications 1 à 30 dans un solvant approprié et un propulseur ainsi qu'un moyen de distribution de ladite composition aérosol.

36. Procédé de traitement des fibres kératiniques, en particulier des cheveux, **caractérisé en ce qu'**on applique sur lesdites fibres la composition telle que définie dans les revendications 1 à 30, avant ou après la mise en forme de la coiffure.

37. Utilisation d'une composition selon l'une quelconque des revendications 1 à 30 dans ou pour la fabrication d'une formulation cosmétique de coiffage.